# EUROPEAN PATENT SPECIFICATION

(11) **EP 3 339 403 B1**
(45) Date of publication and mention of the grant of the patent: **06.02.2019**
(21) Application number: 17203318.5
(22) Date of filing: 23.11.2017
(51) Int. Cl.: C10M 159/24

(54) **MAGNESIUM SULFONATE SYNTHESIS**
MAGNESIUMSULFONATSYNTHESE
SYNTHÈSE DE SULFONATE DE MAGNÉSIUM

(30) Priority: 22.12.2016 EP 16206440
(43) Date of publication of application: 27.06.2018
(73) Proprietor: Infineum International Limited, Abingdon Oxfordshire OX13 6BB (GB)
(72) Inventor: Skinner, Philip, Abingdon, Oxfordshire OX13 6BB (GB); Phillips, Daniel, Abingdon, OX13 6BB (GB); Marsh, Adam, Abingdon, Oxfordshire OX13 6BB (GB); Wilkinson, Thomas Daniel, Abingdon, Oxfordshire OX13 6BB (GB)
(74) Representative: Lewis, Pauline Therese

(56) References cited:
- EP-A1- 0 323 088
- US-A- 4 647 387
- US-A1- 2011 136 711
- US-B1- 6 197 075

## Description

### FIELD OF THE INVENTION

This invention relates to the preparation of overbased magnesium sulfonates such as those useful as detergent additives in lubricating oil compositions (lubricants) for lubricating the crankcase of spark-ignited or compression-ignited internal combustion engines.

### BACKGROUND OF THE INVENTION

Overbased magnesium sulfonates are well known, as is their use as additives in oilbased compositions, for example lubricants, greases and fuels. They function as detergents and acid neutralisers, thereby reducing wear and corrosion and, when used in engines, extending engine life.

The art describes many processes for preparing overbased magnesium sulfonates, preferably involving the carbonation, in the presence of an organic solvent or diluent, of a mixture of an oil-soluble sulfonate and/or an oil-soluble sulfonic acid and an excess of a magnesium compound (e.g. magnesium oxide) above that required to react with any acid present. Processes described have involved various special measures, such as use of particular reaction conditions and/or incorporation of one or more additional substances into the mixture to be carbonated, including, for example, water, alcohols and promoters of various types.

The use of weak acids as promoters is known in the art, which discusses the specific grades of magnesium oxide, as the magnesium compound, that need to be used.

The activity of magnesium oxide (MgO) is typically defined as light-burned, hard-burned or dead-burned (or in similar terms), according to the temperature at which it is formed via a calcination process. Light-burned, usually made at a calcining temperature of 700-1000°C, is considered to have high activity due to its large surface area per unit mass. Dead-burned, usually made at a calcining temperature of 1500-2000°C, is considered to have low activity due to its small surface area per unit mas/s. Hard-burned, usually made at a calcining temperature of 1000-1500°C, has intermediate activity. The "Citric Acid Number", as defined herein, quantifies activity, high numbers indicating low activity and low numbers indicating high activity.

US-A-4,647,387; US-A-4,129,589; and US-A-6,197,075 each describe processes using light-burned MgO, and US-A-5,534,168 describes a process using hard-burned MgO. However, none of these documents describes a process that can be used with either light-burned, hard-burned or dead-burned grades of MgO interchangeably or with mixtures of different grades, to produce an overbased magnesium sulfonate within the same specification.

### SUMMARY OF THE INVENTION

The present invention meets the above problem by employing, in the preparative process, a promoter system comprising, as a first promoter, a polyisobutene succinic anhydride (PIBSA) of defined number average molecular weight, and, as a second promoter, a salicylic acid.

Thus, in a first aspect, the invention provides a process for the preparation of an overbased magnesium sulfonate comprising:
(A) preparing a mixture of a sulfonic acid or salt thereof, a magnesium oxide, water, a C₁ -C₅ alkanol such as methanol, a hydrocarbon solvent and a promoter system comprising a combination of first and second promoters, where
   the first promoter is a polyisobutene succinic anhydride of weight average molecular weight 500 to 1500 gmol⁻¹ which is present in the range of 50 -200, such as 70-170, or 88-145, g per kg of overbased magnesium sulfonate product, and the second promoter is a salicylic acid which is present in the range of 5-20 or 5-13, g per kg of overbased magnesium sulfonate product; and
(B) carbonating the mixture with an acidic gas to form the overbased magnesium sulfonate, as defined in claim 1.

In a second aspect, the invention provides an overbased magnesium sulfonate obtained or obtainable by the process of the above first aspect having a TBN of 390-425 mg KOH g⁻¹, a kinematic viscosity at 100°C of below 300 mm² s⁻¹, and less than 2% sediment.

The examples in this specification demonstrate the flexibility in the sourcing of MgO, to prepare overbased magnesium sulfonates, that is possible by employing the defined promoter system. The inventive process is thus more versatile and has the capability of using MgO from different sources.

### DETAILED DESCRIPTION OF THE INVENTION

### DEFINITIONS

In this specification, the following words and expressions, if and when used, have the meanings given below:
"active ingredients" or "(a.i.)" refers to additive material that is not diluent or solvent;
"comprising" or any cognate word specifies the presence of stated features, steps, or integers or components, but does not preclude the presence or addition of one or more other features, steps, integers, components or groups thereof. The expressions "consists of' or "consists essentially of' or cognates may be embraced within "comprises" or any cognate word. The expression "consists essentially of' permits inclusion of substances not materially affecting the characteristics of the composition to which it applies. The expression "consists of' or cognates means that only the stated features, steps, integers components or groups thereof to which the expression refers are present;
"hydrocarbyl" means a chemical group of a compound that contains hydrogen and carbon atoms and that is bonded to the remainder of the compound directly via a carbon atom. The group may contain one or more atoms other than carbon and hydrogen ("hetero atoms") provided they do not affect the essentially hydrocarbyl nature of the group. Those skilled in the art will be aware of suitable groups (e.g., halo, especially chloro and fluoro, amino, alkoxyl, mercapto, alkylmercapto, nitro, nitroso and sulfoxy). The group may be unsaturated and/or may be polymeric. Preferably, the hydrocarbyl group consists essentially of hydrogen and carbon atoms. More preferably, the hydrocarbyl group consists of hydrogen and carbon atoms. Preferably, the hydrocarbyl group is an aliphatic hydrocarbyl group, such as an alkyl group;
"oil-soluble" or "oil-dispersible", or cognate terms, used herein do not necessarily indicate that the compounds or additives are soluble, dissolvable, miscible, or are capable of being suspended in the oil in all proportions. These terms do mean, however, that they are, for example, soluble or stably dispersible in oil to an extent sufficient to exert their intended effect in the environment in which the oil is employed. Moreover, the additional incorporation of other additives may also permit incorporation of higher levels of a particular additive, if desired;
"ashless" in relation to an additive means the additive does not include a metal;
"ash-containing" in relation to an additive means the additive includes a metal;
"major amount" means in excess of 50 mass % of a composition;
"minor amount" means 50 mass % or less of a composition reckoned as active ingredient of the additive(s);
"effective amount" in respect of an additive means an amount of such an additive in the composition (e.g. an additive concentrate) that is effective to provide, and provides, the desired technical effect;
"ppm" means parts per million by mass, based on the total mass of the composition;
"metal content" of a composition or of an additive component, for example molybdenum content or total metal content of the additive concentrate (i.e. the sum of all individual metal contents), is measured by ASTM D5185;
"TBN" in relation to an additive component or of a composition means total base number (mg KOH g⁻¹) as measured by ASTM D2896;
"kV₁₀₀" means kinematic viscosity at 100°C as measured by ASTM D445;
"HTHS" means High Temperature High Shear at 150°C as measured by - CEC-L-36-A-90.
"phosphorus content" is measured by ASTM D5185;
"sulfur content" is measured by ASTM D2622;
"sulfated ash content" is measured by ASTM D874;
Mₙ means number average molecular weight and for polymeric entities may be determined by gel permeation chromatography;
M_{w} means weight average molecular weight and for polymeric entities may be determined by gel permeation chromatography; in this invention, with reference to the PIBSA, stated M_{w} values are those of the polyisobutene from which the PIBSA is derived;
"dispersity" means M_{w}/Mₙ, (denoted by Ð);
"citric acid number" is the time in seconds required to neutralise, at 22°C, a stirred mixture of 1.7 g of the MgO, 100 ml of water, and 100 ml of a citric acid solution containing 26 g citric acid monohydrate and 0.1 g phenolphthalein in 1 litre of aqueous solution. Neutralisation is indicated by the mixture turning pink. Citric acid number maybe referred to as "CAN" in the text hereof.

Also it will be understood that various components used, essential as well as optimal and customary, may react under condition of formulation, storage and use and that the invention also provides the product(s) obtainable or obtained by any such reaction.

Further it is understood that any upper and lower quantity, range or ratio limits set forth herein may be independently combined.

### PROMOTER SYSTEM

### Polyisobutene succinic anhydride (PIBSA)

The polyisobutene may be prepared by cationic polymerization of isobutene. Common polymers from this class include polyisobutenes obtained by polymerization of a C₄ refinery stream having a butene content of 35 to 75 mass % and an isobutene content of 30 to 60 mass %, in the presence of a Lewis acid catalyst such as aluminum trichloride or boron trifluoride. Polyisobutene is readily available by cationic polymerization from butene streams (e.g., using AlCl₃ or BF₃ catalysts). Such polyisobutenes generally contain residual unsaturation in amounts of one ethylenic double bond per polymer chain, positioned along the chain. One embodiment utilizes polyisobutene prepared from a pure isobutene stream or a Raffinate I stream to prepare reactive isobutene polymers with terminal vinylidene olefins. These polymers, referred to as highly reactive polyisobutene (HR-PIB), may have a terminal vinylidene content of at least 65%. The preparation of such polymers is described, for example, in U.S. Patent No. 4,152,499. HR-PIB is known and HR-PIB is commercially available under the tradenames Glissopal™ (from BASF) and Ultravis™ (from BP-Amoco).

Methods for making polyisobutene are known. Polyisobutene can be functionalized by halogenation (e.g. chlorination), the thermal "ene" reaction, or by free radical grafting using a catalyst (e.g. peroxide), as described below.

To produce the PIBSA, the polymer backbone may be functionalized with succinic anhydride-producing moieties selectively at sites of carbon-to-carbon unsaturation on the polymer or hydrocarbon chains, or randomly along chains using one or more of the three processes mentioned above or combinations thereof, in any sequence.

Processes for reacting polyisobutenes with succinic anhydrides and the preparation of derivatives from such compounds are disclosed in U.S. Patent Nos. 3,087,936; 3,172,892; 3,215,707; 3,231,587; 3,272,746; 3,275,554; 3,381,022; 3,442,808; 3,565,804; 3,912,764; 4,110,349; 4,234,435; 5,777,025; 5,891,953; as well as EP 0 382 450 B1; CA-1,335,895 and GB-A-1,440,219. The polyisobutene may be functionalized with succinic anhydride moieties by reacting the polymer or hydrocarbon under conditions that result in the addition of functional moieties or agents, i.e., acid anhydrides, onto the polymer chains primarily at sites of carbon-to-carbon unsaturation (also referred to as ethylenic or olefinic unsaturation) using the halogen- or radical-assisted functionalization (e.g. chlorination) processes, such as chloro or radical maleation.

Functionalization is preferably accomplished by halogenating, e.g., chlorinating or brominating, the unsaturated α-olefin polymer to 1 to 8, preferably 3 to 7, mass % chlorine or bromine, based on the weight of polymer, by passing the chlorine or bromine through the polymer at a temperature of 60 to 250, preferably 130 to 220, e.g., 140 to 190,°C for about 0.5 to 10, preferably 1 to 7, hours. The halogenated polymer (hereinafter backbone) is then reacted with sufficient reactant capable of adding the required number of functional moieties to the backbone, e.g., monounsaturated carboxylic reactant, at 100 to 250, usually 140 to 220,°C for about 0.5 to 10, e.g., 3 to 8, hours, such that the product obtained will contain the desired number of moles of the carboxylic reactant per mole of the halogenated backbones. Alternatively, the backbone and the carboxylic reactant are mixed and heated while adding chlorine to the hot material.

US 4,234,435 (above-mentioned) describes PIBSA's made by the chloro-route (Diels-Alder process). Its abstract states "carboxylic acid acylating agents are derived from polyalkenes such as polybutenes, and a dibasic, carboxylic reactant such as maleic or fumaric acid or certain derivatives thereof. These acylating agents are characterized in that the polyalkenes from which they are derived have a Mn value of about 1300 to about 5000 and a Mw/Mn value of about 1.5 to about 4. The acylating agents are further characterized by the presence within their structure of at least 1.3 groups derived from the dibasic, carboxylic reactant for each equivalent weight of the groups derived from the polyalkene. The acylating agents can be reacted with a further reactant subject to being acylated such as polyethylene polyamines and polyols (e.g., pentaerythritol) to produce derivatives useful per se as lubricant additives or as intermediates to be subjected to post-treatment with various other chemical compounds and compositions, such as epoxides, to produce still other derivatives useful as lubricant additives."

CA 2,471,534 describes PIBSA's made by the ene-reaction. Its abstract relates to "a process for forming an ene reaction product wherein an enophile, such as maleic anhydride, is reacted with reactive polyalkene having a terminal vinylidene content of at least 30 mol%, at high temperature in the presence of a free radical inhibitor. The polyalkenyl acylating agents are useful per se as additives in lubricating oils, functional fluids, and fuels and also serve as intermediates in the preparation of other products (e.g., succinimides) useful as additives in lubricating oils, functional fluids, and fuels. The presence of the free radical inhibitor during the high temperature reaction results in a reaction product that is low, or substantially free from sediment."

It is believed that the Diels-Alder process produces a dicyclic two-bond attachment of the succinic group to the polybutene. This is structurally rather rigid and keeps the succinic group limited to an imide structure when reacted with a functionalising agent such as a polyamine. On the other hand an ene-reaction (1,5 hydrogen shift reaction) PIBSA has a single-bond link between the succinic group and polybutene, and as such will allow rotation and opening of the succinic group (to di-carboxylic acid) to allow di-amide formation in the right energy conditions (low temperature) and amine excess.

The polymer backbone can be functionalized by random attachment of functional moieties along the polymer chains by a variety of methods. For example, the polymer, in solution or in solid form, may be grafted with the monounsaturated carboxylic reactant, as described above, in the presence of a free-radical initiator. When performed in solution, the grafting takes place at an elevated temperature in the range of 100 to 260, preferably 120 to 240,°C. Preferably, free-radical initiated grafting would be accomplished in a mineral lubricating oil solution containing, e.g., 1 to 50, preferably 5 to 30, mass % polymer based on the initial total oil solution.

The free-radical initiators that may be used are peroxides, hydroperoxides, and azo compounds, preferably those that have a boiling point greater than 100°C and decompose thermally within the grafting temperature range to provide free-radicals. Representative of these free-radical initiators are azobutyronitrile, 2,5-dimethylhex-3-ene-2, 5-bis-tertiary-butyl peroxide and dicumene peroxide. The initiator, when used, typically is used in an amount of between 0.005 and 2% by weight based on the weight of the reaction mixture solution. Typically, the aforesaid monounsaturated carboxylic reactant material and free-radical initiator are used in a weight ratio range of from 1.0:1 to 30:1, preferably 3:1 to 6:1. The grafting is preferably carried out in an inert atmosphere, such as under nitrogen blanketing. The resulting grafted polymer is characterized by having carboxylic acid (or derivative) moieties randomly attached along the polymer chains, it being understood that some of the polymer chains remain ungrafted. The free radical grafting described above can be used for the other polymers and hydrocarbons of the present invention.

The PIBSA, as stated, has a weight average molecular weight of 500-1500 g.mol⁻¹. This may, for example, be 700-1300, such as 900-1150, g.mol⁻¹.

The PIBSA is, as stated, present in the range of 50 -200 g per kg. This may, for example, be such as 70-170, 50-200, such as 88-145, g per kg of overbased magnesium sulfonate product.

### Salicylic Acid (2-hydroxybenzoic acid)

The salicylic acid is, as stated, present in the range of 5 -20 g. This may, for example, 5-13, g per kg of overbased magnesium sulfonate. The salicylic acid may be hydrocarbyl- substituted, such as alkyl-substituted, on the benzene ring, though it is preferably unsubstituted. Also, an acid derivative that is convertible to salicylic acid in the mixture may be used.

### MAGNESEUM OXIDE (MgO)

The MgO may be a light-burn, hard-burn or dead-burn MgO or combinations thereof. In this invention the MgO's are classified as
- "high activity" having a CAN of up to 60 seconds
- "medium activity" having a CAN of from greater than 60 to 200, such as 80 to 200, seconds
- "low activity" having a CAN of from greater than 200 to 700, preferably greater than 200 to 600, seconds.

The conditions of step (B) of the first aspect of the invention may be controlled to optimize the properties of the overbased magnesium sulfonate product, depending in the activity of the MgO, as will be discussed below.

Magnesium oxide is commercially available in various grades as may be indicated in the examples in this specification.

### ACIDIC GAS

This is most preferably carbon dioxide and is described in the art. Carbonation may be carried out as described in the art such as at an elevated temperature.

### SULFONIC ACID OR SALT

This is oil-soluble and may be natural or synthetic. Synthetic alkyl or alkylaryl sulfonates and sulfonic acids with an average molecular weight of 475 g mol⁻¹ and an active ingredient level of approximately 86% are preferred. Such acids and salts are described in the art.

### C₁ - C₅ ALKANOLS

These are water-soluble. Examples are methanol, ethanol, isopropanol, n-propanol, butanol and pentanol, methanol being preferred.

### HYDROCARBON SOLVENT

This is a solvent in which the sulfonic acid and the overbased sulfonate are at least partially soluble, and is used in an amount sufficient to keep the mixture fluid during carbonation. The solvent is advantageously volatile, preferably with a boiling point at atmospheric pressure of below 150° C, so that it can be removed after the completion of carbonation. Examples of suitable hydrocarbon solvents are aliphatic hydrocarbons, for example, hexane or heptane, and aromatic hydrocarbons, for example, benzene, toluene or xylene, the preferred solvent being xylene. Typically, the solvent is used in an amount of 5 parts by mass per part by mass of the magnesium oxide.

### CONDITIONS OF STEP (B)

The magnesium sulfonate product, to be acceptable, is normally required to have a low product viscosity (less than 300 mm² s⁻¹) at a TBN of approximately 390-425 mg KOHg⁻¹ and low levels (less than 2%) of process sediment.

When using certain grades of MgO, the above properties may be achieved by starting step (B) at elevated temperature, increasing to the reflux temperature of the reaction mixture and subsequently cooling. However, when using this temperature profile with, for example, more reactive grades of MgO, it has been found that the above combination of product properties may not be achieved or that no product may be obtained.

It is however found, according to an embodiment of this invention, that any MgO grade, regardless of activity, can be used to make acceptable products by appropriate selection of the temperature (e.g. the carbonation temperature) of step (B). In particular, the following are to be noted:
- When using a "high activity" MgO grade, a product with high process sediment and low TBN is obtained at a carbonation temperature of 60°C. At lower carbonation temperatures, e.g. less than 60 or less than 55, such as 20 to less than 60, °C, the product does not have such debits;
- When using a "medium activity" MgO grade, a product with high process sediment and low TBN is obtained at a carbonation temperature of 40°C and a product with high viscosity is obtained at a carbonation temperature of 70°C. At intermediate temperatures, such as from above 40 to less than 70, such from 45 to 65, °C, the product does not have such debits;
- When using a "low activity" MgO grade, a product with high process sediment is obtained at a carbonation temperature of 60°C. At higher temperatures, such as greater than 60, e.g. 65 or more, such as 65 to 80, °C, the product does not have such a debit.

### LUBRICATING COMPOSITIONS

The overbased magnesium sulfonates of the invention may be used as detergent additives in lubricating compositions.

Lubricating compositions of the invention may be lubricants suitable for use as motor vehicle motor oils comprising a major amount of oil of lubricating viscosity and minor amounts of performance-enhancing additives, including the detergent material. The lubricating composition may also be in the form of an additive concentrate for blending with oil of lubricating viscosity to make a final lubricant. To form a concentrate, the oil of lubricating viscosity is in a concentrate-forming amount such as known in the art.

The oil of lubricating viscosity (sometimes referred to as "base stock" or "base oil") is the primary liquid constituent of a lubricant, into which additives and possibly other oils are blended, for example to produce a final lubricant (or lubricant composition). A base oil, which is useful for making additive concentrates as well as for making lubricating oil compositions therefrom, may be selected from natural oils (vegetable, animal or mineral) and synthetic lubricating oils and mixtures thereof.

Definitions for the base stocks and base oils in this invention are the same as those found in the American Petroleum Institute (API) publication "Engine Oil Licensing and Certification System", Industry Services Department, Fourteenth Edition, December 1996, Addendum 1, December 1998, which categorizes base stocks as follows:
a) Group I base stocks contain less than 90 percent saturates and/or greater than 0.03 percent sulphur and have a viscosity index greater than or equal to 80 and less than 120 using the test methods specified in Table E-1.
b) Group II base stocks contain greater than or equal to 90 percent saturates and less than or equal to 0.03 percent sulphur and have a viscosity index greater than or equal to 80 and less than 120 using the test methods specified in Table E-1.
c) Group III base stocks contain greater than or equal to 90 percent saturates and less than or equal to 0.03 percent sulphur and have a viscosity index greater than or equal to 120 using the test methods specified in Table E-1.
d) Group IV base stocks are polyalphaolefins (PAO).
e) Group V base stocks include all other base stocks not included in Group I, II, III, or IV.

Typically, the base stock has a viscosity preferably of 3-12, more preferably 4-10, most preferably 4.5-8, mm²/s at 100°C.

**Table E-1: Analytical Methods for Base Stock**

| Property | Test Method |
|---|---|
| Saturates | ASTM D 2007 |
| Viscosity Index | ASTM D 2270 |
| Sulphur | ASTM D 2622 |
| | ASTM D 4294 |
| | ASTM D 4927 |
| | ASTM D 3120 |

Other oils of lubricating viscosity that may be included in the lubricating oil composition are detailed as follows.

Natural oils include animal and vegetable oils (e.g. castor and lard oil), liquid petroleum oils and hydro-refined, solvent-treated mineral lubricating oils of the paraffinic, naphthenic and mixed paraffinic-naphthenic types. Oils of lubricating viscosity derived from coal or shale are also useful base oils.

Synthetic lubricating oils include hydrocarbon oils such as polymerized and interpolymerized olefins (e.g. polybutenes, polypropenes, propene-isobutene copolymers, chlorinated polybutenes, poly(1-hexenes), poly(1-octenes), poly(1-decenes)); alkylbenzenes (e.g. dodecylbenzenes, tetradecylbenzenes, dinonylbenzenes, di(2-ethylhexyl)benzenes); polyphenols (e.g. biphenyls, terphenyls, alkylated polyphenols); and alkylated diphenyl ethers and alkylated diphenyl sulfides and the derivatives, analogues and homologues thereof.

Another suitable class of synthetic lubricating oil comprises the esters of dicarboxylic acids (e.g. phthalic acid, succinic acid, alkyl succinic acids and alkenyl succinic acids, maleic acid, azelaic acid, suberic acid, sebasic acid, fumaric acid, adipic acid, linoleic acid dimer, malonic acid, alkylmalonic acids, alkenyl malonic acids) with a variety of alcohols (e.g. butyl alcohol, hexyl alcohol, dodecyl alcohol, 2-ethylhexyl alcohol, ethylene glycol, diethylene glycol monoether, propylene glycol). Specific examples of these esters include dibutyl adipate, di(2-ethylhexyl) sebacate, di-n-hexyl fumarate, dioctyl sebacate, diisooctyl azelate, diisodecyl azelate, dioctyl phthalate, didecyl phthalate, dieicosyl sebacate, the 2-ethylhexyl diester of linoleic acid dimer, and the complex ester formed by reacting one mole of sebacic acid with two moles of tetraethylene glycol and two moles of 2-ethylhexanoic acid.

Esters useful as synthetic oils also include those made from C₅ to C₁₂ monocarboxylic acids and polyols, and polyol ethers such as neopentyl glycol, trimethylolpropane, pentaerythritol, dipentaerythritol and tripentaerythritol.

Unrefined, refined and re-refined oils can be used in the compositions of the present invention. Unrefined oils are those obtained directly from a natural or synthetic source without further purification treatment. For example, a shale oil obtained directly from retorting operations, a petroleum oil obtained directly from distillation or ester oil obtained directly from an esterification process and used without further treatment would be unrefined oils. Refined oils are similar to the unrefined oils except they have been further treated in one or more purification steps to improve one or more properties. Many such purification techniques, such as distillation, solvent extraction, acid or base extraction, filtration and percolation, are known to those skilled in the art. Re-refined oils are obtained by processes similar to those used to obtain refined oils applied to refined oils that have been already used in service. Such re-refined oils are also known as reclaimed or reprocessed oils and often are additionally processed by techniques for treating spent additive and oil breakdown products.

Other examples of base oil are gas-to-liquid ("GTL") base oils, i.e. the base oil may be an oil derived from Fischer-Tropsch synthesised hydrocarbons made from synthesis gas containing H₂ and CO using a Fischer-Tropsch catalyst. These hydrocarbons typically require further processing in order to be useful as a base oil. For example, they may, by methods known in the art, be hydroisomerized; hydrocracked and hydroisomerized; dewaxed; or hydroisomerized and dewaxed.

The oil of lubricating viscosity may also comprise a Group I, Group IV or Group V base stocks or base oil blends of the aforementioned base stocks.

### CO-ADDITIVES

The lubricating oil compositions of all aspects of the present invention may further comprise one or more phosphorus-containing compounds; oxidation inhibitors or antioxidants; dispersants; other metal detergents; and other co-additives, provided they are different from the magnesium sulfonate additives of the invention. These will be discussed in more detail below.

Suitable phosphorus-containing compounds include dihydrocarbyl dithiophosphate metal salts, which are frequently used as antiwear and antioxidant agents. The metal is preferably zinc, but may be an alkali or alkaline earth metal, or aluminum, lead, tin, molybdenum, manganese, nickel or copper. The zinc salts are most commonly used in lubricating oil in amounts of 0.1 to 10, preferably 0.2 to 2, mass %, based upon the total weight of the lubricating oil composition. They may be prepared in accordance with known techniques by first forming a dihydrocarbyl dithiophosphoric acid (DDPA), usually by reaction of one or more alcohol or a phenol with P₂S₅, and then neutralizing the formed DDPA with a zinc compound. For example, a dithiophosphoric acid may be made by reacting mixtures of primary and secondary alcohols. Alternatively, multiple dithiophosphoric acids can be prepared where the hydrocarbyl groups on one are entirely secondary in character and the hydrocarbyl groups on the others are entirely primary in character. To make the zinc salt, any basic or neutral zinc compound could be used but the oxides, hydroxides and carbonates are most generally employed. Commercial additives frequently contain an excess of zinc due to the use of an excess of the basic zinc compound in the neutralization reaction.

The preferred zinc dihydrocarbyl dithiophosphates are oil-soluble salts of dihydrocarbyl dithiophosphoric acids and may be represented by the following formula: 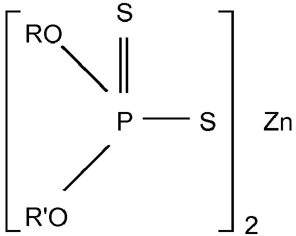 wherein R and R' may be the same or different hydrocarbyl radicals containing from 1 to 18, preferably 2 to 12, carbon atoms and including radicals such as alkyl, alkenyl, aryl, arylalkyl, alkaryl and cycloaliphatic radicals. Particularly preferred as R and R' groups are alkyl groups of 2 to 8 carbon atoms. Thus, the radicals may, for example, be ethyl, n-propyl, i-propyl, n-butyl, i-butyl, sec-butyl, amyl, n-hexyl, i-hexyl, n-octyl, decyl, dodecyl, octadecyl, 2-ethylhexyl, phenyl, butylphenyl, cyclohexyl, methylcyclopentyl, propenyl, butenyl. In order to obtain oil solubility, the total number of carbon atoms (i.e. in R and R') in the dithiophosphoric acid will generally be 5 or greater. The zinc dihydrocarbyl dithiophosphate (ZDDP) can therefore comprise zinc dialkyl dithiophosphates. Lubricating oil compositions of the present invention suitably may have a phosphorus content of no greater than about 0.08 mass % (800 ppm). Preferably, in the practice of the present invention, ZDDP is used in an amount close or equal to the maximum amount allowed, preferably in an amount that provides a phosphorus content within 100 ppm of the maximum allowable amount of phosphorus. Thus, lubricating oil compositions useful in the practice of the present invention preferably contain ZDDP or other zinc-phosphorus compounds, in an amount introducing from 0.01 to 0.08, such as from 0.04 to 0.08, preferably, from 0.05 to 0.08, mass % of phosphorus, based on the total mass of the lubricating oil composition.

Oxidation inhibitors or antioxidants reduce the tendency of mineral oils to deteriorate in service. Oxidative deterioration can be evidenced by sludge in the lubricant, varnish-like deposits on the metal surfaces, and by viscosity growth. Such oxidation inhibitors include hindered phenols, alkaline earth metal salts of alkylphenolthioesters having preferably C₅ to C₁₂ alkyl side chains, calcium nonylphenol sulfide, oil-soluble phenates and sulfurized phenates, phosphosulfurized or sulfurized hydrocarbons or esters, phosphorous esters, metal thiocarbamates, oil- soluble copper compounds as described in U.S. Patent No. 4,867,890, and molybdenum-containing compounds.

Aromatic amines having at least two aromatic groups attached directly to the nitrogen atom constitute another class of compounds that is frequently used for antioxidancy. Typical oil-soluble aromatic amines having at least two aromatic groups attached directly to one amine nitrogen atom contain from 6 to 16 carbon atoms. The amines may contain more than two aromatic groups. Compounds having a total of at least three aromatic groups in which two aromatic groups are linked by a covalent bond or by an atom or group (e.g., an oxygen or sulfur atom, or a -CO-, -SO₂- or alkylene group) and two are directly attached to one amine nitrogen atom are also considered aromatic amines having at least two aromatic groups attached directly to the nitrogen atom. The aromatic rings are typically substituted by one or more substituents selected from alkyl, cycloalkyl, alkoxy, aryloxy, acyl, acylamino, hydroxy, and nitro groups. The amount of any such oil-soluble aromatic amines having at least two aromatic groups attached directly to one amine nitrogen atom should preferably not exceed 0.4 mass %.

A dispersant is an additive whose primary function is to hold solid and liquid contaminations in suspension, thereby passivating them and reducing engine deposits at the same time as reducing sludge depositions. For example, a dispersant maintains in suspension oil-insoluble substances that result from oxidation during use of the lubricant, thus preventing sludge flocculation and precipitation or deposition on metal parts of the engine.

Dispersants in this invention are preferably "ashless", as mentioned above, being non-metallic organic materials that form substantially no ash on combustion, in contrast to metal-containing and hence ash-forming materials. They comprise a long hydrocarbon chain with a polar head, the polarity being derived from inclusion of e.g. an O, P, or N atom. The hydrocarbon is an oleophilic group that confers oil-solubility, having, for example 40 to 500 carbon atoms. Thus, ashless dispersants may comprise an oil-soluble polymeric backbone.

A preferred class of olefin polymers is constituted by polybutenes, specifically polyisobutenes (PIB) or poly-n-butenes, such as may be prepared by polymerization of a C₄ refinery stream.

Dispersants include, for example, derivatives of long chain hydrocarbon-substituted carboxylic acids, examples being derivatives of high molecular weight hydrocarbyl-substituted succinic acid. A noteworthy group of dispersants is constituted by hydrocarbon-substituted succinimides, made, for example, by reacting the above acids (or derivatives) with a nitrogen-containing compound, advantageously a polyalkylene polyamine, such as a polyethylene polyamine. Particularly preferred are the reaction products of polyalkylene polyamines with alkenyl succinic anhydrides, such as described in US-A-3,202,678;-3,154,560; -3,172,892; -3,024,195; -3,024,237, -3,219,666; and -3,216,936, that may be post-treated to improve their properties, such as borated (as described in US-A-3,087,936 and-3,254,025), fluorinated or oxylated. For example, boration may be accomplished by treating an acyl nitrogen-containing dispersant with a boron compound selected from boron oxide, boron halides, boron acids and esters of boron acids.

Preferably, the dispersant, if present, is a succinimide-dispersant derived from a polyisobutene of number average molecular weight in the range of 1000 to 3000, preferably 1500 to 2500, and of moderate functionality. The succinimide is preferably derived from highly reactive polyisobutene.

Another example of dispersant type that may be used is a linked aromatic compound such as described in EP-A-2 090 642.

A detergent is an additive that reduces formation of piston deposits, for example high-temperature varnish and lacquer deposits in engines; it normally has acid-neutralising properties and is capable of keeping finely-divided solids in suspension. Most detergents are based on metal "soaps", that is metal salts of acidic organic compounds.

Detergents generally comprise a polar head with a long hydrophobic tail, the polar head comprising the metal salt of the acidic organic compound. The salts may contain a substantially stoichiometric amount of the metal when they are usually described as normal or neutral salts and would typically have a total base number or TBN at 100 % active mass (as may be measured by ASTM D2896) of from 0 to 80. Large amounts of a metal base can be included by reaction of an excess of a metal compound, such as an oxide or hydroxide, with an acidic gas such as carbon dioxide.

The resulting overbased detergent comprises neutralised detergent as an outer layer of a metal base (e.g. carbonate) micelle. Such overbased detergents may have a TBN at 100 % active mass of 150 or greater, and typically of from 200 to 500 or more.

Suitably, detergents that may be used, additional to the magnesium sulfonates of the invention, include oil-soluble neutral and overbased sulfonates, phenates, sulfurised phenates, thiophosphonates, salicylates and naphthenates and other oil-soluble carboxylates of a metal, particularly alkali metal or alkaline earth metals, e.g. Na, K, Li, Ca and Mg. The most commonly-used metals are Ca and Mg, which may both be present in detergents used in lubricating compositions, and mixtures of Ca and/or Mg with Na. Detergents may be used in various combinations.

Additional additives may be incorporated into the compositions of the invention to enable particular performance requirements to be met. Examples of such additives which may be included in the lubricating oil compositions of the present invention are metal rust inhibitors, viscosity index improvers, corrosion inhibitors, oxidation inhibitors, other friction modifiers, anti-foaming agents, anti-wear agents and pour point depressants. Some are discussed in further detail below.

Friction modifiers and fuel economy agents that are compatible with the other ingredients of the final oil may also be included. Examples of such materials include glyceryl monoesters of higher fatty acids, for example, glyceryl mono-oleate; esters of long chain polycarboxylic acids with diols, for example, the butane diol ester of a dimerized unsaturated fatty acid; and alkoxylated alkyl-substituted mono-amines, diamines and alkyl ether amines, for example, ethoxylated tallow amine and ethoxylated tallow ether amine.

Other known friction modifiers comprise oil-soluble organo-molybdenum compounds. Such organo-molybdenum friction modifiers also provide antioxidant and antiwear credits to a lubricating oil composition. Examples of such oil-soluble organo-molybdenum compounds include dithiocarbamates, dithiophosphates, dithiophosphinates, xanthates, thioxanthates, sulfides, and the like, and mixtures thereof. Particularly preferred are molybdenum dithiocarbamates, dialkyldithiophosphates, alkyl xanthates and alkylthioxanthates.

Additionally, the molybdenum compound may be an acidic molybdenum compound. These compounds will react with a basic nitrogen compound as measured by ASTM test D-664 or D-2896 titration procedure and are typically hexavalent. Included are molybdic acid, ammonium molybdate, sodium molybdate, potassium molybdate, and other alkali metal molybdates and other molybdenum salts, e.g., hydrogen sodium molybdate, MoOCl₄, MoO₂Br₂, Mo₂O₃Cl₆, molybdenum trioxide or similar acidic molybdenum compounds.

Among the molybdenum compounds useful in the compositions of this invention are organo-molybdenum compounds of the formula

Mo(R"OCS₂)₄

and

Mo(R"SCS₂)₄

wherein R" is an organo group selected from the group consisting of alkyl, aryl, aralkyl and alkoxyalkyl, generally of from 1 to 30, preferably 2 to 12, carbon atoms most preferably alkyl of 2 to 12 carbon atoms. Especially preferred are the dialkyldithiocarbamates of molybdenum.

Another group of organo-molybdenum compounds useful in the lubricating compositions of this invention are trinuclear molybdenum compounds, especially those of the formula Mo₃SₖLₙQ_{z} and mixtures thereof wherein the L are independently selected ligands having organo groups with a sufficient number of carbon atoms to render the compound soluble or dispersible in the oil, n is from 1 to 4, k varies from 4 to 7, Q is selected from the group of neutral electron donating compounds such as water, amines, alcohols, phosphines, and ethers, and z ranges from 0 to 5 and includes non-stoichiometric values. At least 21 carbon atoms should be present among all the ligand organo groups, such as at least 25, at least 30, or at least 35, carbon atoms.

Lubricating oil compositions useful in all aspects of the present invention preferably contain at least 10, at least 30, at least 40 and more preferably at least 50, ppm molybdenum. Suitably, lubricating oil compositions useful in all aspects of the present invention contain no more than 1000, no more than 750 or no more than 500, ppm of molybdenum. Lubricating oil compositions useful in all aspects of the present invention preferably contain from 10 to 1000, such as 30 to 750 or 40 to 500, ppm of molybdenum (measured as atoms of molybdenum).

The viscosity index of the base stock is increased, or improved, by incorporating therein certain polymeric materials that function as viscosity modifiers (VM) or viscosity index improvers (VII). Generally, polymeric materials useful as viscosity modifiers are those having number average molecular weights (Mn) of from 5,000 to 250,000, preferably from 15,000 to 200,000, more preferably from 20,000 to 150,000. These viscosity modifiers can be grafted with grafting materials such as, for example, maleic anhydride, and the grafted material can be reacted with, for example, amines, amides, nitrogen-containing heterocyclic compounds or alcohols, to form multifunctional viscosity modifiers (dispersant-viscosity modifiers).

Polymers prepared with diolefins will contain ethylenic unsaturation, and such polymers are preferably hydrogenated. When the polymer is hydrogenated, the hydrogenation may be accomplished using any of the techniques known in the prior art. For example, the hydrogenation may be accomplished such that both ethylenic and aromatic unsaturation is converted (saturated) using methods such as those taught, for example, in U.S. Pat. Nos. 3,113,986 and 3,700,633 or the hydrogenation may be accomplished selectively such that a significant portion of the ethylenic unsaturation is converted while little or no aromatic unsaturation is converted as taught, for example, in U.S. Pat. Nos. 3,634,595; 3,670,054; 3,700,633 and Re 27,145. Any of these methods can also be used to hydrogenate polymers containing only ethylenic unsaturation and which are free of aromatic unsaturation.

Pour point depressants (PPD), otherwise known as lube oil flow improvers (LOFIs) lower the lowest temperature at which the lube flows. Compared with VM, LOFIs generally have a lower number average molecular weight. Like VM, LOFIs can be grafted with grafting materials such as, for example, maleic anhydride, and the grafted material can be reacted with, for example, amines, amides, nitrogen-containing heterocyclic compounds or alcohols, to form multifunctional additives.

In the present invention it may be necessary to include an additive which maintains the stability of the viscosity of the blend. Thus, although polar group-containing additives achieve a suitably low viscosity in the pre-blending stage, it has been observed that some compositions increase in viscosity when stored for prolonged periods. Additives which are effective in controlling this viscosity increase include the long chain hydrocarbons functionalized by reaction with mono- or dicarboxylic acids or anhydrides which are used in the preparation of the ashless dispersants as hereinbefore disclosed.

When lubricating compositions contain one or more of the above-mentioned additives, each additive is typically blended into the base oil in an amount that enables the additive to provide its desired function. Representative effective amounts of such additives, when used in crankcase lubricants, are listed below. All the values listed (with the exception of detergent values because the detergents are used in the form of colloidal dispersants in an oil) are stated as mass percent active ingredient (A.I.).

| ADDITIVE | MASS % (Broad) | MASS % (Preferred) |
|---|---|---|
| Dispersant | 0.1 - 20 | 1 - 8 |
| Metal Detergents | 0.1 - 15 | 0.2 - 9 |
| Corrosion Inhibitor | 0 - 5 | 0 - 1.5 |
| Metal dihydrocarbyl dithiophosphate | 0.1 - 6 | 0.1 - 4 |
| Antioxidant | 0 - 5 | 0.01 - 2.5 |
| Pour Point Depressant | 0.01 - 5 | 0.01 - 1.5 |
| Antifoaming Agent | 0 - 5 | 0.001 - 0.15 |
| Supplemental Antiwear Agents | 0 - 1.0 | 0 - 0.5 |
| Friction Modifier | 0 - 5 | 0 - 1.5 |
| Viscosity Modifier | 0.01 - 10 | 0.25 - 3 |
| Base stock | Balance | Balance |

Preferably, the Noack volatility of the fully-formulated lubricating oil composition (oil of lubricating viscosity plus all additives) is no greater than 18, such as no greater than 14, preferably no greater than 10, mass %. Lubricating oil compositions useful in the practice of the present invention may have an overall sulfated ash content of from 0.5 to 2.0, such as from 0.7 to 1.4, preferably from 0.6 to 1.2, mass %.

It may be desirable, although not essential, to prepare one or more additive concentrates comprising additives (concentrates sometimes being referred to as additive packages) whereby several additives can be added simultaneously to the oil to form the lubricating oil composition. When concentrates are used to make lubricants, they may for example for example be diluted with 3-100, e.g. 5-40, parts by mass of oil of lubricating viscosity per part by mass of concentrate.

### EXAMPLES

The invention will now be described in the following non-limiting examples.

### Preparation of Overbased Magnesium Sulfonate: Example 1 (uncontrolled carbonation temperature)

- To a reactor was added salicylic acid (5.6g), polyisobutene (weight average molecular weight 919-1137 g mol⁻¹) succinic anhydride (124.1 g), methanol (101.1 g) and xylene (533 g). Using a Rushton turbine stirrer, this was mixed at a constant speed (400 rpm) to ensure sufficient agitation. Magnesium oxide (citric acid number 83.4 secs; 162.5 g) was added and the temperature raised to 40 °C at which it was held for 10 minutes.
- Xylene (162 g) and sulfonic acid (265 g; 475 g mol⁻¹ average weight; a.i. 86%), followed by water (142 g), were then added and the mixture heated to 60°C over 15 minutes. Carbon dioxide (155.7 g) was then added over 180-210 minutes.
- Antifoam (3 drops) was added and the mixture diluted with Group I or II mineral oil (319 g).
- The mixture was then distilled under nitrogen while being heated to 140 °C.
- The mixture was cooled and centrifuged (2500 rpm). Remaining solvent was then removed *in vacuo* to give the final overbased magnesium sulfonate product.
- If desired, the product may be further diluted to a target TBN with further Group I or II diluent oil.

The final product had the following properties

| | |
|---|---|
| End of process sediment * | 1.2% |
| TBN / D2896 | 399 mgKOH g⁻¹ |
| kV100/ D445 | 160 mm²s⁻¹ |

| | |
|---|---|
| * End of process sediment is measured, after heating the product to 140 °C, by transferring 100mL of the product into a graduated centrifuge tube which conforms to ASTM/D96. The tube is then spun at 1500 rpm for 10 minutes in a Hermle Z513 centrifuge. The sediment volume at the bottom of the tube is then recorded as a %. | |

The above procedure was repeated with 6.1 g salicylic acid and 145 g of the anhydride (Example 2), and with 13 g salicylic acid and 88 g of the anhydride (Example 3). Moreover the above procedure was applied to different MgO grades of lower (Examples 4-5) and higher (Examples 6-7) activity. The properties of the products obtained are summarized in the table below.

| Example | MgO grade activity | Citric Acid No (seconds) | Sediment (%) | TBN (mg KOH g⁻¹⁾ | kV100 (mm²s⁻¹) |
|---|---|---|---|---|---|
| 1 | Medium | 83.4 | 1.2 | 399 | 160 |
| 2 | Medium | 83.4 | 1.2 | 404 | 162 |
| 3 | Medium | 83.4 | 0.7 | 393 | 227 |
| 4 | Low | 550 | 0.6 | 391 | 121 |
| 5 | Low | >500 | 1.2 | 406 | 146 |
| 6 (ref.) | High | 55.5 | 3.4 | 407 | 271 |
| 7 (ref.) | High | 41.9 | No product obtained | | |

Of the above, examples 1-5 achieved each of the target product parameters:
- Sediment below 2%
- kV100 below 300 mm² s⁻¹
- TBN 390 - 425 mg KOH g⁻¹

Example 6 resulted in high sediment, and no product could be isolated from Example 7 due to solidification of the reaction medium during the process.

### Reference Examples

### Ref 1.1

In this example, the procedure of Example 1 was carried out but using acetic acid instead of salicylic acid.

The results were as follows:

| Example | Sediment (%) | TBN (mg KOH g⁻¹) | kV100 (mm² s⁻¹) |
|---|---|---|---|
| Ref 1.1 | 1.0 | 394 | **403** |

The reference example fell outside of the above target product parameter ranges by virtue of the value indicated in bold. Example Ref 1.1 may be regarded as representative of the art as use of a mixture of acetic acid and PIBSA is referred to in § 0043 of US 2011/136711 A1.

### Example 14 Preparation of Overbased Magnesium Sulfonate (controlled carbonation temperatures)

- To a reactor was added salicylic acid (5.6 g), polyisobutene (weight average molecular weight 919-1137 g mol⁻¹) succinic anhydride (124.1 g), methanol (101.1 g) and xylene (533 g). Using a Rushton turbine stirrer, this was mixed at a constant speed (400 rpm) to ensure sufficient agitation. Magnesium oxide (citric acid number 83.4 secs; 162.5 g) was added and the temperature raised to 40 °C at which it was held for 10 minutes.
- Xylene (162 g) and sulfonic acid (265 g; 475 g mol⁻¹ average weight; a.i. 86%), followed by water (142 g) were then added and the mixture heated to 60 °C over 15 minutes. Carbon dioxide (155.7 g) was then added over 210 minutes, maintaining the reaction temperature at 60 °C throughout.
- Antifoam (3 drops) was added and the mixture diluted with Group I or II mineral oil (319 g).
- The mixture was then distilled under nitrogen while being heated to 140 °C.
- The mixture was cooled and centrifuged (2500 rpm). Remaining solvent was then removed *in vacuo* to give the final overbased magnesium sulfonate product.
- If desired, the product may be further diluted to a target TBN with further Group I or II diluent oil.

To the mixture was then added antifoam (3 drops) and diluted with Group I mineral oil (319 g). The reaction mixture was then distilled under nitrogen while heating to 140°C.

The mixture was then cooled and centrifuged at 2500 rpm. The remaining solvent was then removed from the centrifuged mixture *in vacuo.*

Sediment was determined as above.

The above procedure was repeated using different MgO grades and carbonation temperatures, the results being summarised in the table below, including those for Example 14.

| Example | MgO grade activity | Citric Acid No (Seconds) | Carbonation Temp (°C) | Sediment (%) | TBN (mg KOH. g⁻¹) | kV100 (mm²s⁻¹) |
|---|---|---|---|---|---|---|
| 8 | High | 30.0 | 30 | 1.0 | 392 | 129.9 |
| 9 | High | 30.0 | 40 | 1.0 | 390 | 123.7 |
| 10 | High | 30.0 | 50 | 0.8 | 405 | 145 |
| 11 | High | 30.0 | 60 | **2.8** | **378** | 276 |
| 12 | Medium | 83.4 | 40 | **3.7** | **387** | 122 |
| 13 | Medium | 83.4 | 50 | 1.0 | 396 | 154 |
| 14 | Medium | 83.4 | 60 | 0.8 | 398 | 150 |
| 15 | Medium | 83.4 | 65 | 0.9 | 403 | 162 |
| 16 | Medium | 83.4 | 70 | 1.1 | 394 | **365** |
| 17 | Low | >500 | 60 | **2.4** | 400 | 130 |
| 18 | Low | >500 | 65 | 1.4 | 404 | 146 |
| 19 | Low | >500 | 70 | 1.1 | 405 | 127 |

The figures in bold fall outside of the target product parameters.

Using high activity MgO, target products were obtained at carbonation temperatures of 30, 40 and 50 °C (Examples 8-10) but not at 60 °C (Example 11).

Using medium activity MgO, target products were obtained at 50, 60 and 65 °C (Examples 13-15) but not at 40 or 70 °C (Examples 12, 16).

Using low activity MgO, target products were obtained at 65 and 70 °C (Examples 18, 19) but not at 60 °C (Example 17).

### Further Reference Examples

### Example Ref 1.2

The procedure of Example 10 was repeated using dodecyl succinic anhydride instead of the polyisobutene succinic anhydride. The product had high sediment and low TBN, that fell outside the above target product parameters.

| Example | Sediment (%) | TBN (mg KOH g⁻¹) | kV100 (mm² s¹) |
|---|---|---|---|
| Ref 1.2 | **3.4** | **373** | 63 |

### Example Ref 1.3

The procedure of Example 14 was repeated using polyisobutene succinic anhydride, where the polyisobutene had a weight average molecular weight of 1634-2092 g mol⁻¹ rather than 919-1137 g mol⁻¹. The product had high viscosity that fell outside the above target product parameters.

| Example | Sediment (%) | TBN (mg KOH g⁻¹) | kV100 (mm² s¹) |
|---|---|---|---|
| Ref 1.3 | 1.2 | 407 | **1059** |

## Claims

1. A process for the preparation of an overbased magnesium sulfonate comprising:
(A) preparing a mixture of a sulfonic acid or salt thereof, a magnesium oxide, water, a C₁-C₅ alkanol such as methanol, a hydrocarbon solvent and a promoter system comprising a combination of first and second promoters, where
the first promoter is a polyisobutene succinic anhydride of weight average molecular weight 500 to 1500 g mol⁻¹, which is present in the range of 50-200 g per kg of overbased magnesium sulfonate product, and the second promoter is a salicylic acid, which is present in the range of 5-20 g per kg of overbased magnesium sulfonate product; and
(B) carbonating the mixture with an acidic gas to form the overbased magnesium sulfonate, the temperature of step (B) being from above 40 to less than 70 °C where the magnesium oxide is a medium activity MgO having a citric acid number as measured according to the method disclosed on page 5, paragraph 7 of from greater than 60 to 200 seconds; the temperature of step (B) being greater than 60 °C where the magnesium oxide is a low activity MgO having citric acid number of from greater than 200 to 700 seconds; and the temperature of step (B) being less than 60 °C where the magnesium oxide is a high activity MgO having a citric acid number of up to 60 seconds.

2. The process of claim 1 where the first promoter is present in the range of 70-170, such as 88-145, g per kg of overbased magnesium sulfonate product.

3. The process of claim 1 or claim 2 where the second promoter is present in the range of 5-13 g per kg of overbased magnesium sulfonate product.

4. The process of any of claims 1-3 where the magnesium oxide is a medium activity MgO having a citric acid number of from 80-200 seconds.

5. The process of claim 4 where the temperature of step (B) is from 45 to 65 °C.

6. The process of any of claims 1-3 where the magnesium oxide is a low activity MgO having a citric acid number of from from greater than 200 to 600 seconds.

7. The process of claim 6 where the temperature of step (B) is 65 or more, such as 65 to 80, °C.

8. The process of any of claims 1-3 where the temperature of step (B) is less than 55, such as 20 to less than 55°C where the magnesium oxide is a high activity MgO having a citric acid number of up to 60 seconds.

9. The process of any of claims 1 to 8 where the acidic gas is carbon dioxide.

10. The process of any of claims 1 to 9 where the weight average molecular of polyisobutene succinic anhydride is 700-1300, such as 900-1150, g. mol⁻¹.

11. Overbased magnesium sulfonate obtained or obtainable by the process of any of claims 1-10 having a TBN of 390-425 mg KOH g⁻¹ as measured by ASTM D2896, a kinematic viscosity at 100 °C of below 300, such as below 160, mm²s⁻¹ as measured by ASTM D445 and less than 2% sediment measured by centrifuging product heated to 140 °C in a centrifuge tube (ASTM/D96) as the % volume at the bottom of the tube.

12. A lubricating oil composition comprising a major amount of an oil of lubricating viscosity and, as an additive, a minor amount of an overbased magnesium sulfonate as claimed in claim 11, and one or more co-additives.

13. A lubricating oil concentrate for blending with oil of lubricating viscosity to make a lubricating oil composition of claim 12 comprising, as an additive, an overbased magnesium sulfonate as claimed in claim 11, and a concentrate-forming amount of oil of lubricating viscosity.

14. A method of lubricating the crankcase of a spark-ignited or compression-ignited internal combustion engine in operation of the engine, employing, as the crankcase lubricant, a lubricating oil composition of claim 12.

## Patentansprüche

1. Verfahren zur Herstellung von überbasischem Magnesiumsulfonat, bei dem
(A) eine Mischung von Sulfonsäure oder einem Salz davon, Magnesiumoxid, Wasser, C₁- bis C₅-Alkanol, wie Methanol, Kohlenwasserstofflösungsmittel und einem Promotorsystem hergestellt wird, das eine Kombination aus erstem und zweitem Promotor umfasst, wobei
der erste Promotor ein Polyisobutenbernsteinsäureanhydrid mit einem durchschnittlichen Molekulargewicht von 500 bis 1500 g·mol⁻¹ ist, das im Bereich von 50 bis 200 g pro kg überbasischem Magnesiumsulfonatprodukt vorhanden ist, und wobei der zweite Promotor eine Salicylsäure ist, die im Bereich von 5 bis 20 g pro kg überbasischem Magnesiumsulfonatprodukt vorhanden ist, und
(B) die Mischung mit einem sauren Gas karbonisiert wird, um das überbasische Magnesiumsulfonat zu bilden, wobei die Temperatur von Schritt (B) mehr als 40 bis weniger als 70°C beträgt, wobei das Magnesiumoxid ein MgO mit mittlerer Aktivität mit einer Citronensäurezahl, gemessen nach dem auf Seite 5, Absatz 7 offenbarten Verfahren, von mehr als 60 bis 200 Sekunden ist, die Temperatur von Schritt (B) größer als 60°C ist, wenn das Magnesiumoxid ein MgO mit niedriger Aktivität mit einer Citronensäurezahl von mehr als 200 bis 700 Sekunden ist, und die Temperatur von Schritt (B) weniger als 60°C ist, wenn das Magnesiumoxid ein MgO mit hoher Aktivität mit einer Citronensäurezahl bis zu 60 Sekunden ist.

2. Verfahren nach Anspruch 1, bei dem der erste Promotor im Bereich von 70 bis 170, wie 88 bis 145 g pro kg überbasischem Magnesiumsulfonatprodukt vorhanden ist.

3. Verfahren nach Anspruch 1 oder Anspruch 2, bei dem der zweite Promotor im Bereich von 5 bis 13 g pro kg überbasischem Magnesiumsulfonatprodukt vorhanden ist.

4. Verfahren nach einem der Ansprüche 1 bis 3, bei dem das Magnesiumoxid ein MgO mit mittlerer Aktivität mit einer Citronensäurezahl von 80 bis 200 Sekunden ist.

5. Verfahren nach Anspruch 4, bei dem die Temperatur von Schritt (B) 45 bis 65°C beträgt.

6. Verfahren nach einem der Ansprüche 1 bis 3, bei dem das Magnesiumoxid ein MgO mit niedriger Aktivität mit einer Citronensäurezahl von mehr als 200 bis 600 Sekunden ist.

7. Verfahren nach Anspruch 6, bei dem die Temperatur von Schritt (B) 65°C oder mehr beträgt, wie 65 bis 80°C.

8. Verfahren nach einem der Ansprüche 1 bis 3, bei dem die Temperatur von Schritt (B) weniger als 55, wie 20 bis weniger als 55°C beträgt, wobei das Magnesiumoxid ein MgO mit hoher Aktivität mit einer Citronensäurezahl von bis zu 60 Sekunden ist.

9. Verfahren nach einem der Ansprüche 1 bis 8, bei dem das saure Gas Kohlendioxid ist.

10. Verfahren nach einem der Ansprüche 1 bis 9, bei dem das durchschnittliche Molekulargewicht (Gewichtsmittel) von Polyisobutenbernsteinsäureanhydrid 700 bis 1300, wie 900 bis 1150 g·mol⁻¹ ist.

11. Überbasisches Magnesiumsulfonat, das nach dem Verfahren gemäß einem der Ansprüche 1 bis 10 erhältlich ist, mit einer TBN von 390 bis 425 mg KOH g⁻¹, gemessen gemäß ASTM D2896, einer kinematischen Viskosität bei 100°C unter 300, wie unter 160 mm²s⁻¹, gemessen gemäß ASTM D445, und mit weniger als 2 % Sediment, gemessen durch Zentrifugieren von Produkt, das auf 140°C erwärmt worden ist, in einem Zentrifugenröhrchen (ASTM/D96) als prozentuales Volumen am Boden des Röhrchens.

12. Schmierölzusammensetzung, die eine größere Menge Öl mit Schmierviskosität und als Additiv eine geringere Menge eines überbasischen Magnesiumsulfonats gemäß Anspruch 11 und ein oder mehrere Coadditive umfasst.

13. Schmierölkonzentrat zum Vermischen mit Öl mit Schmierviskosität, um eine Schmierölzusammensetzung gemäß Anspruch 12 herzustellen, die als Additiv ein überbasisches Magnesiumsulfonat nach Anspruch 11 und eine konzentratbildende Menge Öl mit Schmierviskosität umfasst.

14. Verfahren zum Schmieren der Kurbelwanne eines funkengezündeten oder kompressionsgezündeten Verbrennungsmotors im Betrieb des Motors, wobei als Kurbelwannenschmierstoff eine Schmierölzusammensetzung gemäß Anspruch 12 verwendet wird.

## Revendications

1. Procédé de préparation d'un sulfonate de magnésium surbasé comprenant :
(A) la préparation d'un mélange d'un acide sulfonique ou d'un de ses sels, d'un oxyde de magnésium, d'eau, d'un alcanol en C₁ à C₅ tel que le méthanol, d'un solvant hydrocarboné et d'un système de promoteurs comprenant une combinaison d'un premier et d'un second promoteur, où
le premier promoteur est un anhydride succinique de polyisobutène de poids moléculaire moyen en poids de 500 à 1500 g.mol⁻¹, qui est présent dans la plage de 50 à 200 g par kg de produit sulfonate de magnésium surbasé, et le second promoteur est un acide salicylique, qui est présent dans la plage de 5 à 20 g par kg de produit sulfonate de magnésium surbasé ; et
(B) la carbonation du mélange avec un gaz acide pour former le sulfonate de magnésium surbasé, la température de l'étape (B) étant au-dessus de 40 à moins de 70°C où l'oxyde de magnésium est un MgO d'activité moyenne ayant un indice d'acide citrique tel que mesuré selon le procédé décrit sur la page 5, paragraphe 7 de plus de 60 à 200 secondes ; la température de l'étape (B) étant supérieure à 60°C où l'oxyde de magnésium est un MgO de faible activité ayant un indice d'acide citrique de 200 à 700 secondes ; et la température de l'étape (B) étant inférieure à 60°C où l'oxyde de magnésium est un MgO d'activité élevée ayant un indice d'acide citrique de jusqu'à 60 secondes.

2. Procédé selon la revendication 1, où le premier promoteur est présent dans la plage de 70 à 170, tel que 88 à 145, g par kg de produit sulfonate de magnésium surbasé.

3. Procédé selon la revendication 1 ou la revendication 2, où le second promoteur est présent dans la plage de 5 à 13 g par kg de produit sulfonate de magnésium surbasé.

4. Procédé selon l'une quelconque des revendications 1 à 3, où l'oxyde de magnésium est un MgO d'activité moyenne ayant un indice d'acide citrique de 80 à 200 secondes.

5. Procédé selon la revendication 4, où la température de l'étape (B) est de 45 à 65°C.

6. Procédé selon l'une quelconque des revendications 1 à 3, où l'oxyde de magnésium est un MgO de faible activité ayant un indice d'acide citrique de plus de 200 à 600 secondes.

7. Procédé selon la revendication 6, où la température de l'étape (B) est de 65°C ou plus, telle que de 65 à 80°C.

8. Procédé selon l'une quelconque des revendications 1 à 3, où la température de l'étape (B) est de moins de 55, telle que de 20 à moins de 55, °C où l'oxyde de magnésium est un MgO d'activité élevée ayant un indice d'acide citrique de jusqu'à 60 secondes.

9. Procédé selon l'une quelconque des revendications 1 à 8, où le gaz acide est le dioxyde de carbone.

10. Procédé selon l'une quelconque des revendications 1 à 9, où le poids moléculaire moyen en poids de l'anhydride succinique de polyisobutène est de 700 à 1300, tel que de 900 à 1150, g.mol⁻¹.

11. Sulfonate de magnésium surbasé obtenu ou pouvant être obtenu par le procédé selon l'une quelconque des revendications 1 à 10 ayant un IBT de 390 à 425 mg KOH g⁻¹ tel que mesuré par la norme ASTM D2896, une viscosité cinématique à 100°C inférieure à 300, tel qu'inférieure à 160, mm²s⁻¹ telle que mesurée par la norme ASTM D445, et moins de 2 % de sédiment mesuré en centrifugeant le produit chauffé à 140°C dans un tube de centrifugation (ASTM/D96) en tant que % en volume au fond du tube.

12. Composition d'huile lubrifiante comprenant une quantité majeure d'huile de viscosité propre à la lubrification et, comme additif, une quantité mineure d'un sulfonate de magnésium surbasé selon la revendication 11, et un ou plusieurs co-additifs.

13. Concentré d'huile lubrifiante pour le mélange avec une huile de viscosité propre à la lubrification pour constituer une composition d'huile lubrifiante selon la revendication 12 comprenant, comme additif, un sulfonate de magnésium surbasé selon la revendication 11, et une quantité formant un concentré d'huile de viscosité propre à la lubrification.

14. Procédé de lubrification du carter d'un moteur à combustion interne à allumage par étincelle ou à allumage par compression lors du fonctionnement du moteur, employant, comme lubrifiant de carter, une composition d'huile lubrifiante selon la revendication 12.
